(19) Europäisches Patentamt / European Patent Office / Office européen des brevets

(11) **EP 3 801 666 B1**

(12) **EUROPÄISCHE PATENTSCHRIFT**

(45) Veröffentlichungstag und Bekanntmachung des Hinweises auf die Patenterteilung:
**04.03.2026  Patentblatt 2026/10**

(21) Anmeldenummer: **19729726.0**

(22) Anmeldetag: **06.06.2019**

(51) Internationale Patentklassifikation (IPC):
*A61M 60/13* (2021.01)        *A61M 60/178* (2021.01)
*A61M 60/216* (2021.01)        *A61M 60/515* (2021.01)
*A61M 60/546* (2021.01)        *A61M 60/816* (2021.01)

(52) Gemeinsame Patentklassifikation (CPC):
**A61M 60/13; A61M 60/178; A61M 60/216;
A61M 60/515; A61M 60/546; A61M 60/816;**
A61M 2205/3368; A61M 2205/36; A61M 2205/3653;
A61M 2205/52

(86) Internationale Anmeldenummer:
**PCT/EP2019/064800**

(87) Internationale Veröffentlichungsnummer:
**WO 2019/234161 (12.12.2019 Gazette 2019/50)**

(54) **IMPLANTIERTES, VASKULÄRES UNTERSTÜTZUNGSSYSTEM MIT MITTELN ZUR BESTIMMUNG DES FLUID-VOLUMENSTROMS EINES DURCH DIESES FLIESSENDEN FLUIDS**

IMPLANTED VASCULAR SUPPORT SYSTEM WITH MEANS FOR DETERMINING A FLUID VOLUME FLOW THERETRHOUGH

SYSTÈME D'ASSISTANCE VASCULAIRE IMPLANTÉ AVEC MOYENS PERMETTANT DE DÉTERMINER UN DÉBIT VOLUMIQUE DE FLUIDE LE TRAVERSANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018  DE 102018208870**

(43) Veröffentlichungstag der Anmeldung:
**14.04.2021  Patentblatt 2021/15**

(73) Patentinhaber: **Kardion GmbH
70376 Stuttgart (DE)**

(72) Erfinder:
• **BAUMBACH, Hardy
  70376 Stuttgart (DE)**
• **KASSEL, Julian
  75181 Pforzheim (DE)**
• **SCHELLENBERG, Inga
  70191 Stuttgart (DE)**
• **BUDDE, Martina
  76229 Karlsruhe (DE)**
• **SCHLEBUSCH, Thomas Alexander
  71272 Renningen (DE)**

(74) Vertreter: **Gauss, Nikolai et al
Pfiz/Gauss Patentanwälte PartmbB
Tübingerstraße 26
70178 Stuttgart (DE)**

(56) Entgegenhaltungen:
**WO-A2-2012/112378        WO-A2-2014/141284
WO-A2-2014/165635**

## Beschreibung

Die Erfindung betrifft ein implantierbares, vaskuläres Unterstützungssystem. Die Erfindung findet insbesondere Anwendung bei (voll-)implantierten Linksherz-Unterstützungssystemen (LVAD).

Implantierte Linksherz-Unterstützungssysteme (LVAD) existieren hauptsächlich in zwei Ausführungsvarianten. Eine erste verbreitete Ausführungsvariante sind (perkutane) minimalinvasive Linksherz-Unterstützungssysteme. Die zweite verbreitete Ausführungsvariante sind unter der Brustkorböffnung invasiv implantierte apikale Linksherz-Unterstützungssysteme. Bei der genannten ersten Variante wird Blut direkt aus dem linken Ventrikel in die Aorta gefördert, da das (perkutane) minimalinvasive Linksherz-Unterstützungssystem mittig in der Aortenklappe positioniert ist. Bei der genannten zweiten Variante wird das Blut apikal aus dem linken Ventrikel über einen Bypass-Schlauch in die Aorta gefördert.

Die Aufgabe eines kardialen Unterstützungssystems ist die Förderung von Blut. Hierbei hat das sog. Herz-Zeit-Volumen (HZV, üblicherweise angegeben in Liter pro Minute) eine hohe klinische Relevanz. Das Herz-Zeit-Volumen betrifft hierbei mit anderen Worten den Gesamtvolumenstrom an Blut (aus einem Ventrikel), insbesondere vom linken Ventrikel hin zur Aorta. Entsprechend eingängig ist das Bestreben, diesen Parameter als Messwert während des Betriebs eines kardialen Unterstützungssystems zu erheben.

Je nach Unterstützungsgrad, der den Anteil des durch ein Fördermittel wie etwa eine Pumpe des Unterstützungssystems geförderten Volumenstroms zum Gesamtvolumenstrom an Blut vom Ventrikel hin zur Aorta beschreibt, gelangt ein gewisser Volumenstrom über den physiologischen Weg durch die Aortenklappe in die Aorta. Das Herz-zeit-Volumen bzw. der Gesamtvolumenstrom ($Q_{HZV}$) vom Ventrikel hin zur Aorta ist demnach üblicherweise die Summe aus Pumpenvolumenstrom ($Q_p$) und Aortenklappen-Volumenstrom ($Q_a$). Dies lässt sich mit folgendem Zusammenhang ausdrücken:

$$Q_{HZV} = Q_p + Q_a$$

Ein etabliertes Verfahren zur Bestimmung des Herz-Zeit-Volumens ($Q_{HZV}$) im klinischen Umfeld ist die Verwendung von Dilutionsverfahren, die jedoch alle auf einen transkutan eingeführten Katheter setzen und daher nur während einer Herzoperation Herz-Zeit-Volumen-Messdaten liefern können. Da die Erfassung des Herz-Zeit-Volumens ($Q_{HZV}$) durch ein LVAD schwer umzusetzen ist, kann $Q_p$ durch geeignete Komponenten des LVAD erfasst werden. Für hohe Unterstützungsgrade (d. h. $Q_p/Q_{HZV}$) geht $Q_a$ gegen Null, sodass annähernd $Q_p$ als Herz-Zeit-Volumen ($Q_{HZV}$) verwendet werden kann.

Ein etabliertes Verfahren zur Messung des Pumpenvolumenstroms ($Q_p$) ist die Korrelation aus den Betriebsparametern des Unterstützungssystems, vor allem der elektrischen Leistungsaufnahme, eventuell ergänzt um weitere physiologische Parameter wie den Blutdruck. Da diese Verfahren auf statistischen Annahmen und dem zugrunde liegenden Pumpen-Kennfeld des verwendeten LVAD basieren, sind die korrelierten $Q_p$ fehlerbehaftet. Zur Steigerung der Messqualität des Parameters $Q_p$ ist daher die Aufnahme eines Flusssensors wünschenswert.

Die WO 2014/141284 A2 beschreibt eine Nierenpumpe. Eine Kanüle für das Fördern von Blut aus einem Ventrikel eines Herzens in eine Aorta gibt es hier nicht.

Die WO 2012/112378 A2 offenbart ein Blutfluss-Assistenzsystem, das eine Blutpumpe mit einer Kanüle und mit einem Durchflusssensor enthält. Der Durchflusssensor hat ein Heizelement in Form eines Thermistors, der in der Mitte des Durchflussquerschnitts in einem Blutstrom angeordnet ist.

Aus der WO 2014/165635 A2 ist eine implantierbare Blutpumpe mit einem Heizelement und einem Temperatursensor für das Bestimmen einer Blutströmung bekannt.

Aufgabe der Erfindung ist es, ein verbessertes implantierbares, vaskuläres Unterstützungssystem zu schaffen, das die Bestimmung eines Blut-Volumenstroms ermöglicht.

Insbesondere ist es eine Aufgabe der Erfindung, ein implantierbares, vaskuläres Unterstützungssystem zu schaffen, mittels dessen ein Fluid-Volumenstrom in einem mit Blut durchströmten Bereich in einem menschlichen oder tierischen Körper bestimmt werden kann, in dem das vaskuläre Unterstützungssystem implantiert bzw. angeordnet ist.

Die Erfindung wird durch die Ansprüche definiert. Die Verfahren sind nicht Teil der beanspruchten Erfindung. Beschrieben wird die Bestimmung eines Fluid-Volumenstroms durch ein implantiertes, vaskuläres Unterstützungssystem, umfassend folgende Schritte:

a) Ermitteln eines Fluidtemperatur-Parameters im Bereich einer Kanüle des Unterstützungssystems,
b) Betreiben eines Heizelements, das eine Änderung einer Fluidtemperatur in der Kanüle bewirken kann,
c) Ermitteln des Fluid-Volumenstroms unter Verwendung zumindest des Fluidtemperatur-Parameters oder dessen Änderung und zumindest eines Heizelement-Betriebsparameters oder dessen Änderung.

Das vaskuläre Unterstützungssystem ist bevorzugt ein kardiales Unterstützungssystem, besonders bevorzugt ein ventrikuläres Unterstützungssystem. Vorzugsweise dient das Verfahren zur Bestimmung eines Fluid-Volumenstroms durch ein Blutgefäß bzw. durch einen Querschnitt des Blutgefäßes. Bei dem Blutgefäß handelt es sich beispielsweise um die Aorta, insbesondere bei einem Linksherz-Unterstützungssystem, oder um den gemeinsamen Stamm (Truncus pulmonalis) in die beiden Lungenarterien, insbesondere bei einem Rechtsherz-Unterstützungssystem, bevorzugt um die Aorta. Bevorzugt dient das Verfahren zur Bestimmung eines Fluid-Volumenstroms aus einem Ventrikel eines Herzens, insbesondere von einem (linken) Ventrikel eines Herzens hin zur Aorta durch ein (voll-)implantiertes, (links-)ventrikuläres (Herz-)Unterstützungssystem. Bei dem Fluid handelt es sich regelmäßig um Blut. Das Unterstützungssystem ist bevorzugt am Ausgang des linken Ventrikels des Herzens bzw. der linken Herzkammer angeordnet. Besonders bevorzugt ist das Unterstützungssystem in Aortenklappenposition angeordnet.

Das Unterstützungssystem ist bevorzugt so implantiert, dass es sich zumindest teilweise, bevorzugt vollständig oder mit mindestens 50 %, besonders bevorzugt mindestens 85 % oder sogar mindestens 95 % seiner (Außen-)Oberfläche in der Fluidströmung befindet. Weiterhin bevorzugt befindet sich das Unterstützungssystem entlang mindestens 50 %, besonders bevorzugt mindestens 85 % oder sogar mindestens 95 % seiner Länge in der Fluidströmung. Bevorzugt befindet sich ein Ende des Unterstützungssystems, im Bereich dessen bzw. an dem sich der Elektromotor befindet, zumindest teilweise in der Aorta. Weiterhin bevorzugt befindet sich das gegenüberliegende Ende des Unterstützungssystems, im Bereich dessen bzw. an dem sich eine (Einlauf-)Kanüle des Unterstützungssystems befindet, zumindest teilweise in einem (dem linken) Ventrikel des Herzens. Weiterhin bevorzugt ist das Unterstützungssystem mittig in der Aortenklappe platziert, sodass distal Blut aus dem Ventrikel angesaugt und proximal in die Aorta ascendens abgegeben wird. Vorzugsweise ist das Unterstützungssystem zumindest teilweise, bevorzugt vollständig oder mit mindestens 20 %, bevorzugt mindestens 40 %, besonders bevorzugt mindestens 50 % oder sogar mindestens 95 % seiner (Außen-)Oberfläche in einem Blutgefäß, wie etwa einer Arterie, insbesondere der Aorta angeordnet. Besonders bevorzugt ist das Unterstützungssystem so implantiert, dass es sich (vollständig) in der (aszendenten oder deszendenten) Aorta befindet.

Der zu bestimmende Fluid-Volumenstrom ist derjenige, der durch das Unterstützungssystem (selbst) strömt. Dies betrifft mit anderen Worten insbesondere einen Fluid-Volumenstrom der nur durch das Unterstützungssystem selbst hindurch fließt. Bei dem zu bestimmenden Fluid-Volumenstrom handelt es sich in der Regel um den sog. Pumpenvolumenstrom (Formelzeichen $Q_p$), der (nur) den Fluss durch das Unterstützungssystem selbst quantifiziert. Das Verfahren ist insbesondere zur Bestimmung des Pumpenvolumenstroms ($Q_p$) eines (voll-)implantierten (links-)ventrikulären Herzunterstützungssystems (LVAD), insbesondere in Aortenklappenposition und/oder durch das Unterstützungssystem selbst geeignet.

Das Verfahren basiert insbesondere auf (thermisch) anemometrischen (Mess-)Prinzipien zur Flussmessung. Grundprinzip ist dabei, dass ein strömendes Medium einen heißen Körper in Abhängigkeit der Strömungsgeschwindigkeit kühlt. Das Verfahren ermöglich in vorteilhafter Weise, eine kontinuierliche, genaue Messung von $Q_p$ durch in ein LVAD integriertes, auf thermischer Anemometrie basierendes Sensorelement. Mit der hier vorgestellten Lösung kann in vorteilhafter Weise das Herz-Zeit-Volumen (jedenfalls näherungsweise durch $Q_p$) auch außerhalb des OP-Szenarios mit vergleichbarer Qualität wie bei Verwendung eines Dilutionskatheters zur Verfügung gestellt werden.

Die hier vorgeschlagene Lösung zeichnet sich insbesondere durch eine Integration ein oder mehrerer Heizelemente oder ein oder mehrerer Heizelemente und ein oder mehrerer Temperatursensoren in eine Einlaufkanüle eines Unterstützungssystems (VAD) aus. Bei dem Verfahren erfolgt in vorteilhafter Weise eine Berechnung von $Q_p$ aus den gemessenen Spannungsdaten mindestens eines Heizelements und/oder mindestens eines Temperatursensors. Hierbei können insbesondere drei mögliche Wirkprinzipien zur Anwendung kommen, eine Konstantstrom-Anemometrie, eine Konstanttemperatur-Anemometrie oder ein Pulsantwortverfahren.

In Schritt a) erfolgt ein Ermitteln eines Fluidtemperatur-Parameters im Bereich einer Kanüle des Unterstützungssystems. Zum Ermitteln kann beispielhaft ein (separater) Temperatursensor verwendet werden. Alternativ oder kumulativ kann das Ermitteln durch das Heizelement selbst erfolgen. Hierzu kann beispielsweise ein elektrischer Serienwiderstand des Heizelements genutzt werden. Bei dem Fluidtemperatur-Parameter kann es sich um eine (Fluid-)Temperatur, einen Temperatursensor-Strom, ein Temperatursensor-Ausgangs-(Strom-) Signal oder einen (temperaturabhängigen) elektrischen Widerstandswert, insbesondere des Heizelements handeln.

In Schritt a) erfolgt bevorzugt ein Betreiben eines Temperatursensors im Bereich einer Kanüle des Unterstützungssystems. Das Betreiben umfasst insbesondere ein Messen einer Fluidtemperatur und/oder einer Änderung der Fluidtemperatur. Vorzugsweise ist der Temperatursensor auf einer Innenoberfläche oder einer Außenoberfläche der Kanüle angeordnet. Weiterhin bevorzugt können mindestens zwei Temperatursensoren vorgesehen sein. Hierbei kann ein Temperatursensor stromauf und ein weiterer Temperatursensor stromab des Heizelements angeordnet sein.

Die Kanüle ist insbesondere eine Einlaufkanüle, die auch als Ansaugrohr bezeichnet werden kann. Die (Einlauf-)Kanüle ist vorzugsweise so eingerichtet, dass sie im implantierten Zustand Fluid aus einem (linken) Ventrikel eines Herzens hin zu einer Strömungsmaschine des Unterstützungssystems und/oder hin zu der Aorta führen kann.

Besonders bevorzugt ist der Temperatursensor bzw. sind die Temperatursensoren beabstandet zu dem Heizelement angeordnet. Dies ermöglich den Vorteil, dass der Temperatursensor thermisch nicht von dem Heizelement beeinflusst wird, was insbesondere dann vorteilhaft ist, wenn der Temperatursensor einen Referenztemperatursensor darstellt. Als Temperatursensor können Heißleiter, Kaltleiter, Widerstandselemente wie Platin, Halbleiterübergänge oder Thermoelemente zum Einsatz kommen.

Der Temperatursensor oder ein weiterer Temperatursensor kann in das Heizelement eingebracht oder an dem Heizelement angeordnet sein. Wenn mindestens zwei Temperatursensoren vorgesehen sind, ist es hierbei bevorzugt, dass ein Referenztemperatursensor beabstandet von dem Heizelement angeordnet und ein weiterer Temperatursensor in das Heizelement eingebracht oder an dem Heizelement angeordnet ist. Wenn nur ein Temperatursensor vorgesehen ist, ist es hierbei ggf. erforderlich, dass während einer Messung einer Referenztemperatur durch den Temperatursensor das Heizelement ausgeschaltet bzw. nicht in einem Heiz-Zustand betrieben wird. Bevorzugt ist eine Platzierung eines flachen Temperatursensors zwischen Kanülen-Innenwand und Heizelement oder ein Aufsetzen eines Temperatursensors auf das Heizelement. Eine besonders bevorzugte Realisierung ist dabei eine mittige Platzierung des Temperatursensors im Heizbereich des Heizelements. Eine mögliche Realisierungsform wäre auch ein Dreischichtaufbau, wobei zwischen einer unteren und einer mittleren Polyimidfolie ein Heizmäander und zwischen der mittleren und einer oberen Polyimidfolie ein Platindrahtmäander als Temperatursensor platziert ist.

Vorzugsweise wird in Schritt a) eine Referenztemperatur des Fluids bestimmt, insbesondere gemessen. Bevorzugt wird die Referenztemperatur durch einen Referenztemperatursensor bestimmt, der besonders bevorzugt Bestandteil des Unterstützungssystems ist. Der Referenztemperatursensor kann beispielsweise in und/oder an einer (Einlauf-)Kanüle des Unterstützungssystems angeordnet sein. Die Referenztemperatur stellt üblicherweise eine Hintergrundtemperatur des Fluids dar, mit anderen Worten eine Fluidtemperatur, die insbesondere nicht durch das Heizelement und/oder eine Strömungsmaschine des Unterstützungssystems thermisch beeinflusst ist.

In Schritt b) wird ein Heizelement betrieben, das eine Änderung einer Fluidtemperatur in der Kanüle bewirken kann. Dies bedeutet mit anderen Worten insbesondere, dass das Heizelement so eingerichtet und angeordnet ist, das es eine Änderung einer Fluidtemperatur in der Kanüle hervorrufen kann. Hierzu kann das Heizelement direkt innerhalb der Kanüle bzw. auf einer Innenoberfläche der Kanüle angeordnet sein. Es ist jedoch (alternativ) möglich, dass das Heizelement in einer Wandung der Kanüle, auf einer Außenoberfläche der Kanüle oder sogar beabstandet von der Kanüle angeordnet ist, solange das Heizelement beispielsweise durch Wärmeleitung dazu in der Lage ist, eine Fluidtemperatur zumindest eines Teils des innerhalb der Kanüle befindlichen Fluids zu erhöhen. Zum Betreiben wird das Heizelement in der Regel mit einem Strom angesteuert.

Vorzugsweise wird das Heizelement mit mindestens einem Heizfilament bzw. Thermofilament gebildet. Bevorzugt ist ein insbesondere rundes bzw. rohrförmiges Heizelement, das die Innenoberfläche der Kanüle zumindest in einem Segmentbereich bzw. Umfangsabschnitt und/oder Längenabschnitt auskleidet. Weiterhin bevorzugt ist das Heizelement in der Art einer (flexiblen) Heizfolie gebildet, die die Innenoberfläche des Kanüle zumindest teilweise auskleidet. Besonders bevorzugt ist in oder an der Folie mindestens ein Heizfilament angeordnet. Vorzugsweise erstreckt sich das Heizfilament (beispielsweise mäanderförmig und/oder in Schleifen) insbesondere durchgängig über mindestens 50 % der oder sogar die (gesamte) von der Folie ausgekleidete(n) Innenoberfläche der Kanüle. Es können mindestens zwei Heizfilamente vorgesehen sein. Bevorzugt ist es, wenn das Heiz- bzw. Thermofilament inwandig in der Kanüle (auf der Innenseite der Kanülenwand) realisiert ist, wodurch in vorteilhafter Weise ein definiertes Blutvolumen untersucht und eine Erhitzung z. B. der Aortenklappe bei Verrutschen des Unterstützungssystems ausgeschlossen werden kann. Wenn mehr als ein Heizelement bzw. Heizfilament vorgesehen ist, können diese auf einander gegenüberliegenden Positionen der Innenoberfläche der Kanüle angeordnet sein. Weiterhin bevorzugt werden die Heizelemente bzw. Heizfilamente gemeinsam angesteuert bzw. bestromt.

Es ist auch vorteilhaft, wenn das Heizelement selbst als Temperatursensor eingesetzt wird. Vorzugsweise ist das Heizelement dazu eingerichtet, dass es sowohl eine Änderung einer Fluidtemperatur in der Kanüle bewirken als auch eine Änderung einer Fluidtemperatur in der Kanüle erfassen, insbesondere messen kann. Insbesondere durch eine geeignete Wahl des Heizelements, insbesondere des Heizfilament-Materials (Widerstandsänderung bei Temperaturänderung) kann das Heizelement selbst als Temperatursensor verwendet werden. Eine vorteilhafte Ausführung des Heizelements ist daher beispielsweise ein (Platin-)Draht-Mäander (mäanderförmig angeordnetes Heizfilament aus einer Platinlegierung) zwischen beispielsweise Polyimid-Folien oder an einer Folie. Vorzugsweise weist das Heizelement im Dünnschichtverfahren hergestellte Heizmäander aus leitfähigen, widerstandsbehafteten Materialien (z. B. Platinlegierung) auf. Hierbei kann das Heizelement beispielhaft dadurch als Temperatursensor eingesetzt werden, dass ein Heizelement-(Serien-)Widerstand gemessen wird. Zur Messung der Referenztemperatur bzw. Fluidhintergrundtemperatur kann der Heizelement-(Serien-)Widerstand beispielsweise bei ausgeschaltetem Heizer bzw. in einer Phase, in der das Heizelement nicht in einem Heiz-Zustand (z. B. bestimmt durch eine Heiz-Spannung und/oder einen Heiz-Strom) betrieben wird, gemessen werden. Wenn das Heizelement selbst als Temperatursensor eingesetzt werden kann, muss kein (weiterer bzw. separater) Temperatursensor vorgesehen sein und in Schritt a) kann in diesem Fall das Heizelement statt dem (separaten) Temperatursensor betrieben werden. In diesem Zusammenhang ist es besonders bevorzugt, dass als Heizelement bzw. in dem Heizelement (nur) ein (Platin-)Heizmäander zum Einsatz kommt, der auch als Temperatursensor genutzt werden kann. Im ausgeschalteten Zustand, d. h. wenn das Heizelement nicht in einem Heiz-Zustand betrieben wird, könnte das (Platin-)Heizelement bzw. der Heizmäander als Referenztemperatursensor genutzt werden, im Betrieb, d. h. wenn das Heizelement in einem Heiz-Zustand betrieben wird, als Heizelement und gleichzeitig als Betriebstemperatursensor. Hierzu kann beispielsweise eine (bekannte) Temperaturabhängigkeit eines Heizelement-(Serien-)Widerstand genutzt werden.

Das Heizelement ist hier eine regelmäßig zusätzlich zu einem Elektromotor des Unterstützungssystems vorgesehene Komponente, die insbesondere getrennt von dem Elektromotor angeordnet ist. Unter einem Heizelement wird hier insbesondere eine elektrisch betreibbare Komponente verstanden, die vorzugsweise mindestens 70%, besonders bevorzugt mindestens 80% oder sogar mindestens 90% der ihr zugeführten elektrischen Energie in Wärme umsetzt. Folglich wird unter einem Heizelement hier insbesondere kein Elektromotor verstanden, der eine Strömungsmaschine des Unterstützungssystems antreibt.

In Schritt c) erfolgt ein Ermitteln des Fluid-Volumenstroms unter Verwendung zumindest des Fluidtemperatur-Parameters oder dessen Änderung und zumindest eines Heizelement-Betriebsparameters oder dessen Änderung. In Schritt c) erfolgt bevorzugt ein Ermitteln des Fluid-Volumenstroms unter Verwendung zumindest eines Temperatursensor-Betriebsparameters oder dessen Änderung und zumindest eines Heizelement-Betriebsparameters oder dessen Änderung. Dies bedeutet mit anderen Worten insbesondere, dass das Ermitteln des Fluid-Volumenstroms unter Verwendung sowohl eines Temperatursensor-Betriebsparameters oder dessen Änderung als auch eines Heizelement-Betriebsparameters oder dessen Änderung erfolgt. Unter einem Heizelement-Betriebsparameter kann beispielsweise eine Heizelement-Temperatur, ein Heizelement-Strom oder ein Heizelement-Ausgangs-(Strom-)Signal verstanden werden. Unter einem Temperatursensor-Betriebsparameter kann eine damit gemessene Temperatur, ein Temperatursensor-Strom oder ein Temperatursensor-Ausgangs-(Strom-)Signal verstanden werden. Unter einer Änderung kann hier insbesondere ein Puls verstanden werden, der vorteilhafterweise von dem Heizelement ausgesendet und von dem Temperatursensor erfasst werden kann.

Das Heizelement wird mit einer definierten elektrischen Leistung betrieben. Hierbei wird eine Temperatur des Heizelements gemessen. Diese (erste) Ausgestaltung betrifft insbesondere eine sog. Konstantstrom-Anemometrie. Bei der Konstantstrom-Anemometrie wird das Heizelement mit definierter elektrischer Leistung betrieben und die resultierende Temperatur wird gemessen.

Nach einer (zweiten) vorteilhaften Ausgestaltung wird vorgeschlagen, dass das Heizelement auf einer konstanten Temperatur gehalten wird. Hierbei kann eine elektrische Leistung des Heizelements gemessen werden. Diese (zweite) Ausgestaltung betrifft insbesondere eine sog. Konstanttemperatur-Anemometrie. Bei der Konstanttemperatur-Anemometrie wird das Heizelement auf konstanter Temperatur gehalten und die dafür notwendige elektrische Leistung wird gemessen.

Nach einer (dritten) vorteilhaften Ausgestaltung wird vorgeschlagen, dass das Heizelement gepulst betrieben wird. Hierbei kann in Schritt c) eine Änderung einer Fluidtemperatur mittels eines insbesondere stromabwärts des Heizelements platzierten Temperatursensors detektiert werden. Diese (dritte) Ausgestaltung betrifft insbesondere ein sog. Pulsantwortverfahren. Beim Pulsantwortverfahren wird das Heizelement gepulst betrieben und es wird die Zeit gemessen, bis der thermische Impuls an einem stromabwärts platzierten Temperatursensor gemessen wird. Zur Verbesserung der Messauflösung kann der gepulste Betrieb durch z. B. eine binäre Zufallszahlenfolge erfolgen und die Zeitverzögerung durch einen Autokorrelator ermittelt werden. Weiterhin bevorzugt ist eine zusätzliche Berücksichtigung der maximalen Amplitude des Antwortpulses in der Berechnung.

Bevorzugt wird der in Schritt c) ermittelte Fluid-Volumenstrom beispielsweise in einem Schritt d) als Regelparameter für das Unterstützungssystem bereitgestellt. Eine Verarbeitungseinheit des Unterstützungssystems kann diesen Regelparameter als Ausgangsgröße, insbesondere einer Steuereinheit des Unterstützungssystems, das vorzugsweise die Leistung eines Elektromotors und damit insbesondere auch die (Blut-)Förderleistung des Unterstützungssystems regelt, bereitstellen.

Nach einem weiteren Aspekt wird eine Verarbeitungseinheit vorgeschlagen, eingerichtet zur Durchführung eines hier vorgeschlagenen Verfahrens. Die Verarbeitungseinheit kann einen Speicher aufweisen, in dem Kalibrierdaten hinterlegt sein können. Alternativ oder zusätzlich zu den Kalibrierdaten kann in dem Speicher auch mindestens ein (geschwindigkeitsabhängiger) Kalibierfaktor und/oder ein thermisches Modell des Heizelements hinterlegt sein. Darüber hinaus kann die Verarbeitungseinheit einen Mikroprozessor umfassen, der auf den Speicher zugreifen kann. Die Verarbeitungseinheit empfängt vorzugsweise Daten von mindestens einem Heizelement und/oder mindestens einem Temperatursensor. Die Verarbeitungseinheit kann weiterhin eine Elektronik-Baugruppe zur Ansteuerung und zum Auslesen des Heizelements und des Temperatursensors umfassen.

Nach einem weiteren Aspekt wird ein implantierbares, vaskuläres Unterstützungssystem vorgeschlagen, umfassend:

- eine Temperaturmesseinrichtung im Bereich einer Kanüle des Unterstützungssystems,
- ein Heizelement, das eine Änderung einer Fluidtemperatur in der Kanüle bewirken kann.

Bei dem Unterstützungssystem handelt es sich vorzugsweise um ein linksventrikuläres Herzunterstützungssystem (LVAD) bzw. ein perkutanes, minimalinvasives Linksherz-Unterstützungssystem. Weiterhin bevorzugt ist dieses voll-implantierbar. Das bedeutet mit anderen Worten insbesondere, dass die zur Erfassung erforderlichen Mittel, insbesondere der Referenztemperatursensor, der Motortemperatursensor und der Stromsensor sich vollständig im Körper des Patienten befinden und dort verbleiben. Besonders bevorzugt ist das Unterstützungssystem so eingerichtet bzw. dazu geeignet, dass es zumindest teilweise in einem Ventrikel, bevorzugt dem linken Ventrikel eines Herzens und/oder einer Aorta, insbesondere in Aortenklappenposition angeordnet werden kann.

Die Temperaturmesseinrichtung ist bevorzugt mit einem Temperatursensor gebildet. Weiterhin bevorzugt kann die Temperaturmesseinrichtung auch einen weiteren Temperatursensor umfassen. Es ist jedoch nicht zwingend, dass die Temperaturmesseinrichtung separat zu dem Heizelement vorgesehen ist. Vielmehr kann die Temperaturmesseinrichtung auch in dem Heizelement und/oder durch das Heizelement selbst gebildet sein. Besonders bevorzugt ist hierzu eine (implizite) Temperaturmessung über einen Heizelement-Serienwiderstand.

Weiterhin bevorzugt umfasst das Unterstützungssystem eine Strömungsmaschine, wie etwa eine Pumpe. Bevorzugt hat das Unterstützungssystem einen Elektromotor. Der Elektromotor ist dabei regelmäßig ein Bestandteil der Strömungsmaschine. Das Unterstützungssystem ist vorzugsweise länglich und/oder schlauchartig gebildet. Bevorzugt sind eine (Einlauf-)Kanüle und eine Strömungsmaschine im Bereich einander gegenüberliegender Enden des Unterstützungssystems angeordnet.

Das Unterstützungssystem umfasst weiterhin eine Verarbeitungseinheit, eingerichtet zur Durchführung eines hier vorgeschlagenen Verfahrens.

Die im Zusammenhang mit dem Verfahren erörterten Details, Merkmale und vorteilhaften Ausgestaltungen können entsprechend auch bei der hier vorgestellten Verarbeitungseinheit und/oder dem Unterstützungssystem auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und/oder Erkenntnissen aus anderen Figuren und/oder der vorliegenden Beschreibung zu kombinieren. Es zeigen schematisch:

Fig. 1a     ein perkutanes, minimalinvasives Linksherz-Unterstützungssystem

Fig. 1b     ein unter der Brustkorböffnung invasiv implantiertes Linksherz-Unterstützungssystem,

Fig. 2      ein implantiertes, vaskuläres Unterstützungssystem, das ein Konstantstrom- und Konstanttemperatur-Verfahren ausüben kann,

Fig. 3      eine Komponentenarchitektur eines Unterstützungssystems gemäß Fig. 2,

Fig. 4      eine Veranschaulichung eines Regelkreises eines Unterstützungssystems gemäß Fig. 2,

Fig. 5      ein weiteres implantiertes, vaskuläres Unterstützungssystem, das ein Konstantstrom- und Konstanttemperatur-Verfahren ausüben kann,

Fig. 6      ein weiteres implantiertes, vaskuläres Unterstützungssystem, das ein Pulsantwortverfahren ausüben kann,

Fig. 7      ein weiteres implantiertes, vaskuläres Unterstützungssystem, das ein Pulsantwortverfahren ausüben kann, und

Fig. 8      zeitliche Messwertverläufe zu dem Unterstützungssystem gemäß Fig. 6 oder Fig. 7.

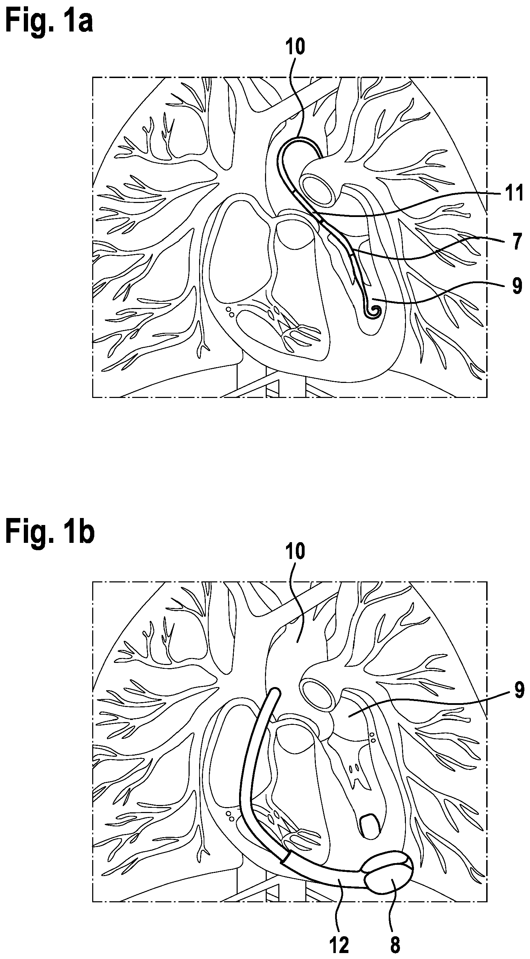

Implantierte Linksherz-Unterstützungssysteme (LVAD) existieren hauptsächlich in zwei Ausführungsvarianten, wie sie in den Fig. 1a und 1b gezeigt sind. Fig. 1a zeigt ein (perkutanes) minimalinvasives Linksherz-Unterstützungssystem 7, während Fig. 1b ein unter der Brustkorböffnung invasiv implantiertes apikales Linksherz-Unterstützungssystem 8 zeigt. Die Variante nach Fig. 1a fördert Blut direkt aus dem linken Ventrikel 9 in die Aorta 10, da das (perkutane) minimalinvasive Linksherz-Unterstützungssystem 7 mittig in der Aortenklappe 11 positioniert ist. Die Variante nach Fig. 1b fördert das Blut apikal aus dem linken Ventrikel 9 über einen Bypass-Schlauch 12 in die Aorta 10.

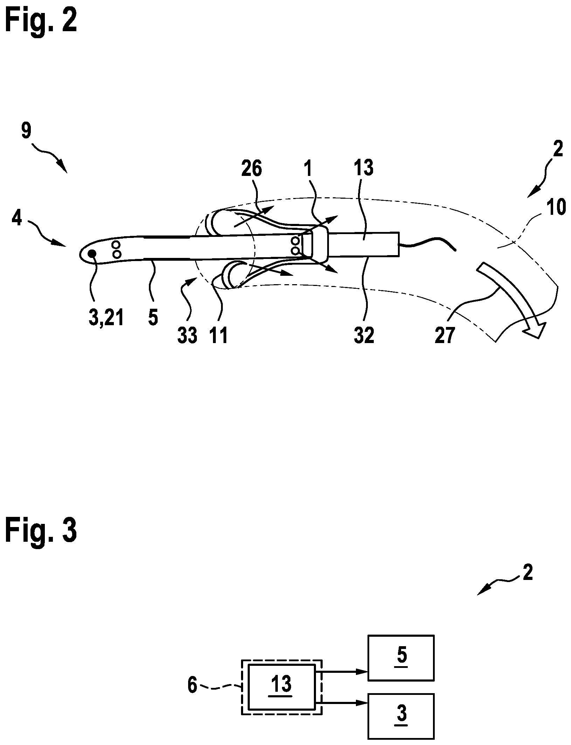

Fig. 2 zeigt schematisch ein implantiertes, vaskuläres Unterstützungssystem 2 in Aortenklappenposition, das ein Konstantstrom- und Konstanttemperatur-Verfahren ausüben kann.

Das Unterstützungssystem 2 ist hier beispielhaft ein linksventrikuläres Herzunterstützungssystem (LVAD). schlauchartige längliche Struktur mit einem Kanülenabschnitt, in dem eine (Einlauf-)Kanüle 4 ausgebildet ist, und mit einem an den Kanülenabschnitt angeschlossenen Strömungsmaschinenabschnitt, in dem eine Strömungsmaschine 32 angeordnet ist. Das Unterstützungssystem 2 ragt aus der Aorta 10 durch die Aortenklappen 11 distal in den Ventrikel 9. Die (Einlauf-)Kanüle 4 des Unterstützungssystems 2 ragt dabei in den Ventrikel 9 hinein. Durch die Kanüle 4 wird ein Fluid-Volumenstrom 1 unter Verwendung der Strömungsmaschine 32 (etwa einer Pumpe, die einen Elektromotor aufweisen kann) des Unterstützungssystems 2 aus dem Ventrikel 9 in die Aorta 10 gefördert, beispielsweise gepumpt. Daher wird der Fluid-Volumenstrom 1 auch als Pumpenvolumenstrom ($Q_p$) bezeichnet, der nur den Fluss durch das Unterstützungssystem 2 selbst quantifiziert.

Zudem ist in Fig. 2 zu erkennen, dass ein gewisser Aortenklappen-Volumenstrom 26 über den physiologischen Weg durch die Aortenklappen 11 in die Aorta 10 gelangt. Das Herz-Zeit-Volumen bzw. der im Bereich des Unterstützungssystems 2 durch eine Querschnittsgeometrie 33 der Aorta 10 hindurchtretende Fluid-Gesamtvolumenstrom 27 ($Q_{HZV}$) vom Ventrikel 9 hin zur Aorta 10 ist demnach die Summe aus Fluid-Volumenstrom 1 ($Q_p$) und Aortenklappen-Volumenstrom 26 ($Q_a$).

Im Bereich der Kanüle 4 ist ein Temperatursensor 3 angeordnet. Hierzu ist der Temperatursensor 3 beispielhaft am distalen Ende der Kanüle 4 (im Ventrikel 9, dort von wo das Fluid, etwa Blut, zuströmt) platziert. Ferner weist das Unterstützungssystem 2 ein Heizelement 5 auf, das eine Änderung einer Fluidtemperatur in der Kanüle 4 bewirken kann, z. B. durch Joul'sche Wärme bzw. Ohm'sche Widerstandserwärmung, wenn das Heizelement 5 bestromt wird.

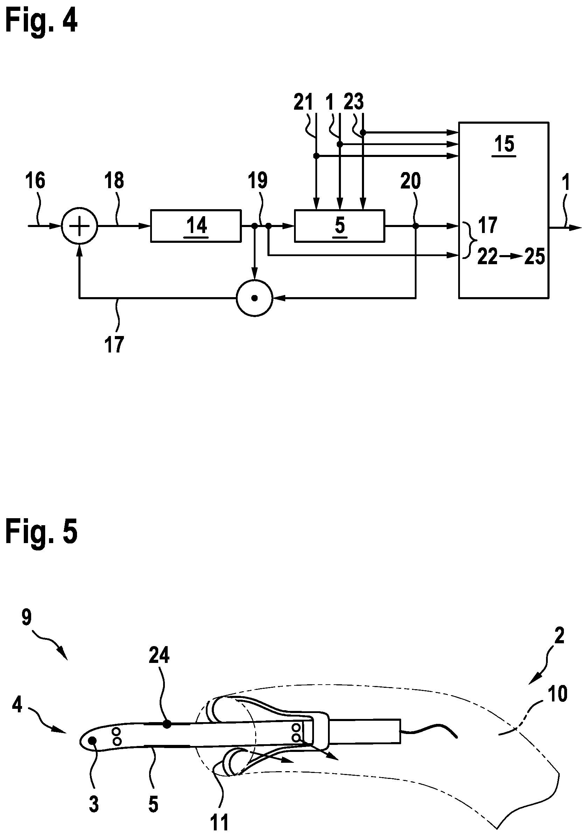

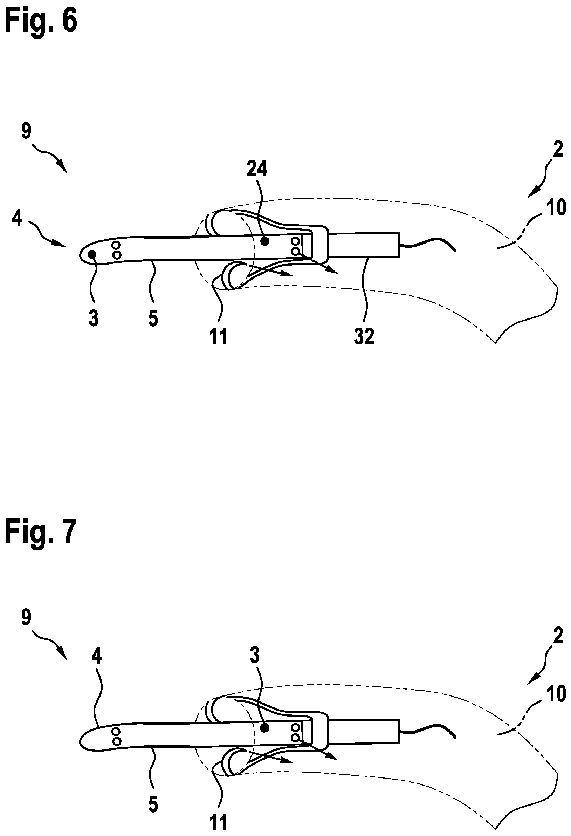

Bei dem Temperatursensor 3 gemäß Fig. 2 handelt es sich um einen Referenz-Temperatursensor, der eine Referenztemperatur 21 erfasst, die hier beispielhaft die Hintergrund-Bluttemperatur ist. Dazu ist der (Referenz-)Temperatursensor 3 im thermisch unbeeinflussten Blutfluss vor dem eine Wärmequelle darstellenden Heizelement 5 platziert, hier beispielhaft im Bereich vor dem bzw. stromauf des Heizelement(s) 5. Statt separatem (Referenz-)Temperatursensor 3 kann auch der Wert eines weiteren (zweiten), z. B. auf Höhe des Heizelements 5 oder stromab davon angeordneten Temperatursensors (vgl. Fig. 5, 6: Bezugszeichen 24; Fig. 7.: Bezugszeichen 3) genutzt werden, wenn das System nicht in Betrieb ist und somit dieser weitere Temperatursensor nicht von dem Heizelement 5 beeinflusst wird. Da sich die Bluttemperatur bei ruhendem Patienten nur langsam ändert, kann dieser Wert ebenfalls eine gute Abschätzung für die Hintergrundtemperatur darstellen. Darüber hinaus kann je nach Gestaltung des Heizelements 5 auch der elektrische Widerstand des Heizelements 5 selbst als Temperatursensor 3 verwendet werden.

Wird ein separater Referenz-Temperatursensor genutzt, wie dies nach der Darstellung gemäß Fig. 2 mit dem Temperatursensor 3 der Fall ist, sollte dieser derart im Unterstützungssystem 2 positioniert sein, dass er nicht durch die Wärmeabgabe des Heizelements 5 beeinflusst wird, beispielsweise an der hin zum Ventrikel 9 weisenden Spitze des Unterstützungssystems 2 bzw. der Kanüle 4 und/oder thermisch entkoppelt stromaufwärts (des Blutflusses) vom Heizelement 5. Dadurch wird es in vorteilhafter Weise möglich, den Temperaturhub durch thermische Energiezufuhr in das beobachtete Fluidvolumen zu bestimmen. Aufgrund der gerichteten Strömung im Medium bestimmt sich ein beispielhafter Mindestabstand des Referenztemperatursensors zum Heizelement 5 insbesondere (hauptsächlich) aus der Wärmeleitfähigkeit des Trägermaterials. Vorteilhaft sind Abstände von mindestens 5 mm [Millimeter] bei nichtmetallischem Trägermaterial.

Das Wirkprinzip beruht hier darauf, bei hinreichend bekannter thermischer Kapazität (Formelzeichen C; vgl. Bezugszeichen 23 in Fig. 4) des Fluids, hier Blutes, die elektrische Leistung dQ zu bestimmen, die zur Erwärmung des Bluts um eine definierte Temperatur dT notwendig ist:

$$C = \frac{\mathrm{d}Q}{\mathrm{d}T}$$

Bei hinreichend bekannter Wärmekapazität C (wird im Algorithmus vorgehalten), gemessenem Energiezufluss dQ und aus zwei gemessenen (Fluid-)Temperaturen ermitteltem Temperaturhub dT kann somit das im Beobachtungszeitrum umgesetzte Flüssigkeitsvolumen V bzw. der Fluid-Volumenstrom 1 (Formelzeichen Q) berechnet werden. Die für die Differenz dT erforderliche Hintergrund-Bluttemperatur kann dabei entweder über einen (Referenz-)Temperatursensor 3 oder aus dem Wert eines weiteren Temperatursensors (vgl. obige Ausführungen) berechnet werden, wenn das Heizelement ausreichend lange nicht aktiv war.

Das Heizelement 5 ist hier beispielhaft mit einem Heizfilament bzw. Thermofilament gebildet. Das Thermofilament ist inwandig in der Kanüle 4, die auch als Ansaugrohr bezeichnet werden kann, realisiert, wodurch in vorteilhafter Weise ein definiertes Blutvolumen untersucht und eine Erhitzung z. B. der Aortenklappe 11 bei Verrutschen des Unterstützungssystems ausgeschlossen werden kann.

Zur Betriebsweise der Ausführung nach Fig. 2 wird auch die nachstehenden Ausführungen zur Fig. 4 verwiesen.

Fig. 3 zeigt schematisch eine Komponentenarchitektur eines Unterstützungssystems gemäß Fig. 2. Das Unterstützungssystem 2 umfasst hier beispielhaft ein Steuergerät 13, einen Temperatursensor 3 sowie ein beispielhaft als Thermo- bzw. Heizfilament ausgebildetes Heizelement 5. Das Steuergerät 13 ist hier beispielhaft ein Bestandteil einer Verarbeitungseinheit 6 des Unterstützungssystems 2.

Fig. 4 zeigt schematisch eine Veranschaulichung eines Regelkreises eines Unterstützungssystems 2 gemäß Fig. 2. Die Bezugzeichen werden einheitlich verwendet, sodass zur Erläuterung der Betriebsweise der Ausführungsform gemäß den Figuren 2 bis 4 hier auch auf die Figuren 2 und 3 Bezug genommen wird.

Der in Fig. 4 gezeigte beispielhafte Regelkreis kann in dem Steuergerät 13 nach Fig. 3 implementiert sein, das wiederum ein Bestandteil des Unterstützungssystems 2, insbesondere einer Verarbeitungseinheit 6 des Unterstützungssystems 2 sein kann. Der Regelkreis umfasst einen Regler 14 und das Heizelement 5. Die das Heizelement 5 (Regelstrecke) beeinflussenden Störgrößen sind die Referenztemperatur 21, der Fluid-Volumenstrom 1 und die Wärmekapazität 23 (des Fluids, hier Blutes). Die Regelgröße ist hier der Strom 20 und wird an den Regler 14 zurückgeführt. Dabei erfolgt eine gemeinsame Rückführung des Stroms 20 (Regelgröße) und der Spannung 19 (Stellgröße) durch die ermittelte Ist-Leistung 17. Die Regelabweichung 18 ergibt sich aus einer Subtraktion der Ist-Leistung 17 von der Soll-Leistung 16. Die genannten Störgrößen Fluid-Volumenstrom 1, Referenztemperatur 21 und Wärmekapazität 23 sowie der Strom 20 (Regelgröße) und die Spannung 19 (Stellgröße) werden zudem einer Berechnungseinheit 15 bereitgestellt, die aus der Spannung 19 und dem Strom 20 die Ist-Leistung 17 und den elektrischen Ist-Widerstand 22 des Heizelements 5 ermittelt und zudem aus dem elektrischen Ist-Widerstand 22 die Heizelementtemperatur 25 ermittelt (z. B. aufgrund der bekannten Temperaturabhängigkeit des Widerstands). Die Berechnungseinheit 15 berechnet daraus den Fluid-Volumenstrom 1, wobei dieser als gemittelter Volumenstrom bereitgestellt werden kann.

Bei der Ausführung als Konstantstrom-Anemometrie wird das Heizelement 5 hier beispielhaft von dem Regler 14 im Steuergerät 13 mit konstanter Leistung beaufschlagt und sowohl der elektrische Widerstand 22 zur Messung der Heizelementtemperatur 25, als auch die Referenztemperatur 21 aus dem Referenz-Temperatursensor 3 ausgelesen (bzw. Heizelementwiderstand 22 bei ausgeschaltetem Heizer (d. h. das Heizelement 5 nicht in einem Heiz-Zustand betrieben) zur Ermittelung der Referenztemperatur 21). In der Berechnungseinheit 15 findet die Berechnung des Fluid-Volumenstroms 1 bzw. von $Q_p$ auf Basis der elektrischen Heizelementleistungsaufnahme 17, der auf Basis des elektrischen Widerstands 22 des Heizelements 5 ermittelten Heizelementtemperatur 25 und der Referenztemperatur 21 statt.

Bei der Ausführung als Konstanttemperatur-Anemometrie wird die Heizelementtemperatur 25 des Heizelements 5 hier beispielhaft durch den Regler 14 auf einer definierten Temperatur oder auf einem definierten Temperaturhub über der Referenz- bzw. Hintergrundtemperatur 21 gehalten. Auf Basis der dafür notwendigen Filamentleistungsaufnahme 17 und der Hintergrundtemperatur 21 wird in der Berechnungseinheit 15 des Steuergeräts 13 der Fluid-Volumenstrom 1 bzw. $Q_p$ berechnet.

Fig. 5 zeigt schematisch ein weiteres implantiertes, vaskuläres Unterstützungssystem 2, das ein Konstantstrom- und Konstanttemperatur-Verfahren ausüben kann. Das Unterstützungssystem 2 nach Fig. 5 hat viele Gemeinsamkeiten mit dem Unterstützungssystem 2 gemäß Fig. 2, sodass insoweit auf die obigen Ausführungen zur Fig. 2 Bezug genommen wird. Die Ausführungsvariante nach Fig. 5 unterscheidet sich darin von der nach Fig. 2, dass ein weiterer (zweiter) Temperatursensor 24 thermisch an das Heizelement 5 angekoppelt platziert ist, sodass die Temperaturermittlung des Heizelements 5 nicht über den elektrischen Widerstand 22 des Heizelements 5, sondern über den elektrischen Widerstand des weiteren Temperatursensors 24 stattfinden kann.

Fig. 6 zeigt schematisch ein weiteres implantiertes, vaskuläres Unterstützungssystem 2, das ein Pulsantwortverfahren ausüben kann. Bei dieser Variante wird ein in der Kanüle 4 bevorzugt inwandig angeordneter weiterer Temperatursensor 24 räumlich vom Heizelement 5 abgesetzt (in Richtung der Strömungsmaschine 32, stromabwärts des Heizelements 5), sodass Laufzeit- und thermische Verdünnungseffekte beobachtet werden können. Wie bei den vorhergehend beschriebenen Ausführungsvarianten ist ein optionaler (vgl. Fig. 7) Referenztemperatursensor, der hier durch den Temperatursensor 3 gebildet ist, stromaufwärts platziert, um die Referenz- bzw. Hintergrundtemperatur 21 des Fluids (hier: Bluts) zu bestimmen. Dabei ist hier darauf geachtet, dass der Temperatursensor 3 und der weitere Temperatursensor 24 thermisch vom Heizelement 5 entkoppelt sind und der weitere Temperatursensor 24 aufgrund seiner räumlichen Nähe zur Strömungsmaschine 32 ebenfalls von dieser thermisch entkoppelt ist. Je nach Trägermaterial sind hier 5-10 mm Abstand gute Werte.

Das Heizelement 5 wird mit einem Leistungspuls 31 beaufschlagt und bringt eine definierte Energiemenge $E_p$ in das Blutvolumen der Kanüle 4 ein, was zu einer Erhöhung der Bluttemperatur führt. Durch die (Pumpen-)Aktivität der Strömungsmaschine 32 strömt das Blut mit einer $Q_p$-abhängigen Strömungsgeschwindigkeit weiter in Richtung des weiteren Temperatursensors 24, der nach einer $Q_p$-abhängigen Laufzeit $\Delta t$ ein Temperaturmaximum $T_m$ beobachtet. Auf Basis von $E_p$ oder der Heizelementleistungsaufnahme 17 wird mit $\Delta t$, der Referenztemperatur 21 und $T_m$ im Steuergerät 13 der Fluid-Volumenstrom 1 bzw. $Q_p$ berechnet (Laufzeit $\Delta t$ oder Laufzeit $\Delta t$ und Amplitudenhöhe $T_m$).

Die beobachtbaren Effekte sind sowohl eine Laufzeit, wobei ein hoher Fluid-Volumenstrom 1 einer kurzen Laufzeit vom Heizelement 5 zum weiteren Temperatursensor 24 entspricht, als auch, aufgrund des festen thermischen Widerstands vom Heizelement 5 zum Blutvolumen und der festen thermischen Kapazität 23 des Blutes, eine Amplitudenänderung, wobei eine langsame Fluid-Volumenströmung 1 einer starken Temperaturerhöhung am weiteren Temperatursensor 24 und eine hohe Strömung einer nur kleinen Temperaturerhöhung entspricht.

Fig. 7 zeigt schematisch ein weiteres implantiertes, vaskuläres Unterstützungssystem 2, das ein Pulsantwortverfahren ausüben kann. Das Unterstützungssystem 2 nach Fig. 7 hat viele Gemeinsamkeiten mit dem Unterstützungssystem 2 gemäß Fig. 6, sodass insoweit auf die obigen Ausführungen zur Fig. 6 Bezug genommen wird. Der Unterschied besteht darin, dass in Fig. 7 nur ein Temperatursensor 3 vorgesehen ist. Dieser ist bevorzugt innwandig in der Kanüle 4 erfüllt hier den Zweck, den der weitere Temperatursensor 24 in der Ausführungsform nach Fig. 6 erfüllt. Somit kommt die Ausführungsform gemäß Fig. 7 ohne (separaten) Referenztemperatursensor aus.

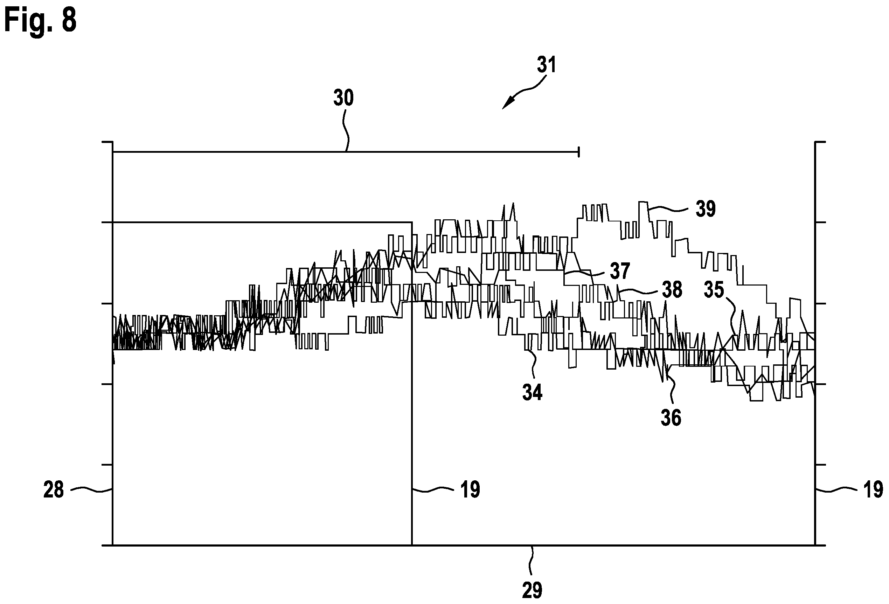

Fig. 8 zeigt schematisch zeitliche Messwertverläufe zu dem Unterstützungssystem 2 gemäß Fig. 6 bzw. Fig. 7. Hierbei sind mittels des stromab des Heizelements 5 angeordneten Temperatursensors (Bezugszeichen 24 in Fig. 6 und Bezugszeichen 3 in Fig. 7) gemessene Temperaturverläufe über der Zeit 29 aufgetragen, wobei die Temperatur als Spannungswert über einen Analog-Digital-Wandler gemessen wurde, sodass sowohl die Spannung 19, als auch ein Analog-Digital-Umsetzer-Ausgang 28 über der Zeit 29 aufgetragen sind. Es sind diverse Messwertverläufe eingetragen, nämlich ein erster Messwertverlauf 34, ein zweiter Messwertverlauf 35, ein dritter Messwertverlauf 36, ein vierter Messwertverlauf 37, ein fünfter Messwertverlauf 38 sowie ein sechster Messwertverlauf 39, wobei die Messwertverläufe nach absteigendem Fluid-Volumenstrom (Pumpenvolumenfluss) geordnet sind, Messwertverlauf 39 also den Temperaturverlauf am Temperatursensor bei geringem und Messwertverlauf 34 den Temperaturverlauf am Temperatursensor bei hohem Fluid-Volumenstrom repräsentiert. Zudem ist lediglich exemplarisch die Zeitdifferenz 30 bis der Puls 31 des Messwertverlaufs 39 gemessen wurde markiert. Deutlich zu erkennen ist, dass die Zeitdifferenz 30 invers proportional zum Fluid-Volumenstrom ist, ebenso die Amplitude (das Maximum) des Messwertverlaufs. Darüber hinaus sind in der Darstellung nach Fig. 8 auch die Pulse 31 der weiteren Messwertverläufe 34, 35, 36, 37 und 38, insgesamt also sechs Pulse 31 zu erkennen. Zur Erläuterung der Messwertverläufe wird auf die vorstehenden Ausführungen zu den Fig. 6 und 7, insbesondere zu den dort beschriebenen, beobachteten Effekten verwiesen.

Die hier vorgeschlagene Lösung ermöglicht insbesondere einen oder mehrere der nachstehenden Vorteile:

- Durch Integration des Sensors in die Einlaufkanüle des VAD wird ein Gewebekontakt zum Heizelement verhindert, wodurch eine Gewebeschädigung verhindert wird.
- Die Integration in die Einlaufkanüle hat den Vorteil, dass die (durchströmte) Geometrie und damit das untersuchte Blutvolumen bekannt ist, was die Kalibrierung des Sensors je nach Realisierungsvariante vereinfacht bzw. ersetzt. Marktübliche Katheter erfordern die Gabe eines Eiswasser-Bolus, um auf das Blutgefäßvolumen zu kalibrieren.

- Eine kontinuierliche $Q_p$-Messung ermöglicht die schnelle Diagnose von suction, d. h. einem Ansaugen des Einlaufschlauches an die Ventrikelwand, wodurch die Pumpenfunktion beeinträchtigt wird.

Zusammenfassend sind insbesondere folgende bevorzugte Merkmale der Erfindung festzuhalten:
Ein Verfahren zur Bestimmung eines Fluid-Volumenstroms 1 durch ein implantiertes, vaskuläres Unterstützungssystem 2, umfasst folgende Schritte:

a) Ermitteln eines Fluidtemperatur-Parameters im Bereich einer Kanüle (4) des Unterstützungssystems (2),
b) Betreiben eines Heizelements (5), das eine Änderung einer Fluidtemperatur in der Kanüle (4) bewirken kann,
c) Ermitteln des Fluid-Volumenstroms (1) unter Verwendung zumindest des Fluidtemperatur-Parameters oder dessen Änderung und zumindest eines Heizelement-Betriebsparameters oder dessen Änderung.

Ein imlantierbares, d.h. in dem menschlichen oder tierischen Körper anordenbares vaskuläres Unterstützungssystem enthält eine Temperaturmesseinrichtung im Bereich einer Kanüle 4 des Unterstützungssystems 2 und weist ein Heizelement 5 auf, das eine Änderung einer Fluidtemperatur in der Kanüle (4) bewirken kann.

## Patentansprüche

1. Implantierbares, vaskuläres Unterstützungssystem (2)

mit einer schlauchartigen länglichen Struktur mit einem Kanülenabschnitt,
in dem eine Kanüle (4) für das Fördern von Blut aus einem Ventrikel eines Herzens in eine Aorta ausgebildet ist,
mit einem an den Kanülenabschnitt angeschlossenen Strömungsmaschinenabschnitt, in dem eine Strömungsmaschine (32) angeordnet ist, die für das Fördern von Blut aus einem Ventrikel eines Herzens durch die Kanüle (4) in eine Aorta dient,
mit einem Temperatursensor (3) für das Messen einer Referenztemperatur (21), der an einem der Strömungsmaschine (32) abgewandten Ende der Kanüle (4) angeordnet ist,
mit einem Heizelement (5), das in der Kanüle (4) zwischen dem Temperatursensor (3) und der Strömungsmaschine (32) angeordnet ist, das eine Änderung einer Fluidtemperatur in der Kanüle (4) bewirken kann,

mit einem Steuergerät (13), in dem ein Regelkreis implementiert ist, der einen Regler (14) enthält, und

mit einer Verarbeitungseinheit (6), die eine Berechnungseinheit (15) enthält, die einen Blut-Volumenstrom $Q_p$ durch die Kanüle (4) auf Basis zumindest eines Heizelement-Betriebsparameters oder dessen Änderung und einer mittels des Temperatursensors (3) gemessenen Referenztemperatur (21) bestimmt.

2. Unterstützungssystem nach Anspruch 1, wobei die Berechnungseinheit (15) den Blut-Volumenstrom $Q_p$ durch die Kanüle (4) unter Verwendung eines Heizelement-Betriebsparameters in Form einer Temperatur des Heizelements (5) oder in Form eines Temperaturhubs über der mittels des Temperatursensors (3) ermittelten Temperatur und unter Verwendung eines Heizelement-Betriebsparameters in Form einer elektrischen Leistungsaufnahme des Heizelements (5) bestimmt.

3. Unterstützungssystem nach Anspruch 1 oder 2, wobei das Heizelement (5) in der Kanüle (4) in einem von dem Temperatursensor (3) und der Strömungsmaschine (32) beabstandet angeordneten Abschnitt der Kanüle (4) angeordnet ist und wobei das Heizelement (5) eine Änderung einer Fluidtemperatur in der Kanüle (4) bewirken kann, wenn durch das Heizelement (5) ein elektrischer Strom geführt wird, so dass dieses eine Ohm'sche Widerstandserwärmung erfährt.

4. Unterstützungssystem nach einem der Ansprüche 1 bis 3, wobei der Regler (14) das Heizelement (5) mit einer konstanten Leistung beaufschlagt und die Berechnungseinheit (15) eine ermittelte Temperatur (25) des Heizelements (5) erhält.

5. Unterstützungssystem (2) nach einem der Ansprüche 1 bis 4, wobei die Berechnungseinheit (15) eine auf Basis des elektrischen Widerstands (22) des Heizelements (5) ermittelte Temperatur (25) des Heizelements (5) erhält.

6. Unterstützungssystem nach Anspruch 1, wobei der Regler (14) das Heizelement (5) auf einer definierten Temperatur oder auf einem definierten Temperaturhub über der mittels des Temperatursensors (3) ermittelten Temperatur hält und die Berechnungseinheit (15) als ein Heizelement-Betriebsparameter die elektrische Leistungsaufnahme des Heizelements (5) erhält.

7. Unterstützungssystem nach Anspruch 1 oder 6, wobei für das Erfassen der Temperatur des Heizelements (5) ein an das Heizelement (5) thermisch angekoppelter weiterer Temperatursensor (24) vorgesehen ist.

8. Unterstützungssystem nach einem der Ansprüche 1 bis 7, wobei der Temperatursensor (3) an einer Innenwand der Kanüle (4) angeordnet ist.

9. Implantierbares, vaskuläres Unterstützungssystem (2)

mit einer schlauchartigen länglichen Struktur mit einem Kanülenabschnitt, in dem eine Kanüle (4) für das Fördern von Blut aus einem Ventrikel eines Herzens in eine Aorta ausgebildet ist,

mit einem an den Kanülenabschnitt angeschlossenen Strömungsmaschinenabschnitt, in dem eine Strömungsmaschine (32) angeordnet ist, die für das Fördern von Blut aus einem Ventrikel eines Herzens durch die Kanüle (4) in eine Aorta dient,

mit einem Temperatursensor (24, 3), der zwischen einem in der Kanüle (4) angeordneten Heizelement (5) und der Strömungsmaschine (32) von dem Heizelement (5) räumlich abgesetzt angeordnet ist,

wobei das Heizelement (5) eine Änderung einer Fluidtemperatur in der Kanüle (4) bewirken kann, wenn durch das Heizelement (5) ein elektrischer Strom geführt wird, so dass dieses eine Ohm'sche Widerstandserwärmung erfährt,

mit einer Einrichtung für das Beaufschlagen des Heizelements (5) mit einem Leistungspuls, wodurch eine definierte Energiemenge Ep in das Blutvolumen der Kanüle (4) eingebracht wird, und

mit einer Einrichtung für das Messen der Temperatur des durch die Kanüle (4) strömenden Bluts mittels des Temperatursensors (24, 3), und

mit einer Einrichtung für das Berechnen des Volumenstroms Qp auf Basis einer erfassten Laufzeit $\Delta t$ für ein Temperaturmaximum der mittels des Temperatursensors (24, 3) erfassten Temperatur

oder

mit einer Einrichtung für das Berechnen des Volumenstroms Qp auf Basis einer erfassten Laufzeit $\Delta t$ für ein Temperaturmaximum Tm der mittels des Temperatursensors (24, 3) erfassten Temperatur und einer Amplitudenhöhe des Temperaturmaximums Tm.

10. Unterstützungssystem nach Anspruch 9, **dadurch gekennzeichnet, dass** die Temperaturmesseinrichtung einen weiteren Temperatursensor (3) für das Messen einer Referenztemperatur aufweist, der an einem der Strömungsmaschine abgewandten Ende der Kanüle (4) angeordnet ist.

11. Unterstützungssystem nach einem der Ansprüche Anspruch 1 bis 10, **dadurch gekennzeichnet, dass** das Heizelement (5) als ein Heizfilament oder Thermofilament ausgebildet ist.

12. Unterstützungssystem nach einem der Ansprüche 1 bis 11, wobei das Heizelement (5) an einer Innenwand der Kanüle (4) angeordnet ist.

**Claims**

1. Implantable vascular support system (2), comprising

   a tubular, elongate structure
   a cannula portion in which a cannula (4) is designed for pumping blood from a ventricle of a heart into an aorta,
   a turbomachine portion connected to the cannula portion, in which a turbomachine (32) is arranged for pumping blood from a ventricle of a heart through the cannula (4) into an aorta,
   a temperature sensor (3) for measuring a reference temperature (21), which is arranged at one end of the cannula (4) facing away from the turbomachine (32),
   a heating element (5) which is arranged in the cannula (4) between the temperature sensor (3) and the turbomachine (32) and can cause a change in the fluid temperature in the cannula (4), a control unit (13) in which a control loop, which includes a controller (14), is implemented, and
   a processing unit (6) containing a calculating unit (15) which determines a volumetric flow $Q_p$ of blood through the cannula (4) on the basis of at least one heating element operating parameter or a change thereof, and on the basis of a reference temperature (21) measured by means of the temperature sensor (3).

2. Support system according to claim 1, wherein the calculating unit (15) determines the volumetric flow $Q_p$ of blood through the cannula (4) using a heating element operating parameter in the form of a temperature of the heating element (5) or in the form of a temperature rise above the temperature determined by means of the temperature sensor (3) and using a heating element operating parameter in the form of an electrical power consumption of the heating element (5).

3. Support system according to claim 1 or 2, wherein the heating element (5) in the cannula (4) is arranged in a portion of the cannula (4) spaced apart from the temperature sensor (3) and the turbomachine (32), and wherein the heating element (5) can cause a change in the fluid temperature in the cannula (4) when an electric current is passed through the heating element (5) so that it experiences ohmic resistance heating.

4. Support system according to any of claims 1 to 3, wherein the controller (14) supplies the heating element (5) with a constant power and the calculating unit (15) receives a determined temperature (25) of the heating element (5).

5. Support system (2) according to any of claims 1 to 4, wherein the calculating unit (15) receives a temperature (25) of the heating element (5) determined on the basis of the electrical resistance (22) of the heating element (5).

6. Support system according to claim 1, wherein the controller (14) maintains the heating element (5) at a defined temperature or at a defined temperature rise above the temperature determined by means of the temperature sensor (3), and the calculating unit (15) receives the electrical power consumption of the heating element (5) as a heating element operating parameter.

7. Support system according to claim 1 or 6, wherein an additional temperature sensor (24) thermally coupled to the heating element (5) is provided for detecting the temperature of the heating element (5).

8. Support system according to any of claims 1 to 7, wherein the temperature sensor (3) is arranged on an inner wall of the cannula (4).

9. Implantable vascular support system (2), comprising

   a tubular, elongate structure
   a cannula portion in which a cannula (4) is designed for pumping blood from a ventricle of a heart into an aorta,
   a turbomachine portion connected to the cannula portion, in which a turbomachine (32) is arranged for pumping blood from a ventricle of a heart through the cannula (4) into an aorta,
   a temperature sensor (24, 3) which is spatially separated from the heating element (5) and arranged between a heating element (5) arranged in the cannula (4) and the turbomachine (32), wherein the heating element (5) can cause a change in the fluid temperature in the cannula (4) when an electric current is passed through the heating element (5) so that it experiences ohmic resistance heating,
   a device for applying a power pulse to the heating element (5), as a result of which a defined amount of energy Ep is introduced into the blood volume of the cannula (4), and

a device for measuring the temperature of the blood flowing through the cannula (4) by means of the temperature sensor (24, 3), and

a device for calculating the volumetric flow Qp on the basis of a detected runtime $\Delta t$ for a temperature maximum of the temperature detected by means of the temperature sensor (24, 3)

or

a device for calculating the volumetric flow Qp on the basis of a detected runtime $\Delta t$ for a temperature maximum Tm of the temperature detected by means of the temperature sensor (24, 3) and an amplitude level of the temperature maximum Tm.

10. Support system according to claim 9, **characterized in that** the temperature measuring device has an additional temperature sensor (3) for measuring a reference temperature, which sensor is arranged at one end of the cannula (4) facing away from the turbomachine.

11. Support system according to any of claims 1 to 10, **characterized in that** the heating element (5) is designed as a heating filament or a thermofilament.

12. Support system according to any of claims 1 to 11, wherein the heating element (5) is arranged on an inner wall of the cannula (4).


**Revendications**

1. Système d'assistance (2) vasculaire implantable

   comportant une structure allongée en forme de tube
   comportant une section de canule
   dans laquelle une canule (4) est réalisée pour le transport de sang depuis un ventricule d'un cœur dans une aorte,
   comportant une section de turbomachine raccordée à la section de canule et dans laquelle est disposée une turbomachine (32) qui sert à transporter du sang depuis un ventricule d'un cœur, à travers la canule (4) et dans une aorte,
   comportant un capteur de température (3) pour la mesure d'une température de référence (21) qui est disposé à une extrémité de la canule (4) opposée à la turbomachine (32),
   comportant un élément chauffant (5) qui est disposé dans la canule (4) entre le capteur de température (3) et la turbomachine (32) et qui peut provoquer une modification d'une température de fluide dans la canule (4), comportant un appareil de commande (13) dans lequel est mis en oeuvre un circuit de régulation qui comprend un moyen de régulation (14), et comportant une unité de traitement (6) qui comprend une unité de calcul (15) qui détermine un débit volumique de sang $Q_p$ à travers la canule (4) sur la base d'au moins un paramètre de fonctionnement d'élément chauffant ou de sa modification et d'une température de référence (21) mesurée au moyen du capteur de température (3).

2. Système d'assistance selon la revendication 1, dans lequel l'unité de calcul (15) détermine le débit volumique de sang $Q_p$ à travers la canule (4) en utilisant un paramètre de fonctionnement d'élément chauffant sous la forme d'une température de l'élément chauffant (5) ou sous la forme d'une élévation de température au-dessus de la température déterminée au moyen du capteur de température (3) et en utilisant un paramètre de fonctionnement d'élément chauffant sous la forme d'une consommation de puissance électrique de l'élément chauffant (5).

3. Système d'assistance selon la revendication 1 ou 2, dans lequel l'élément chauffant (5) est disposé dans la canule (4) dans une section de la canule (4) disposée à distance du capteur de température (3) et de la turbomachine (32) et dans lequel l'élément chauffant (5) peut provoquer une modification d'une température de fluide dans la canule (4) lorsqu'un courant électrique est passé à travers l'élément chauffant (5), de sorte que celui-ci subit un chauffage par résistance ohmique.

4. Système d'assistance selon l'une des revendications 1 à 3, dans lequel le moyen de régulation (14) applique une puissance constante à l'élément chauffant (5) et l'unité de calcul (15) obtient une température (25) déterminée de l'élément chauffant (5).

5. Système d'assistance (2) selon l'une des revendications 1 à 4, dans lequel l'unité de calcul (15) obtient une température (25) de l'élément chauffant (5) déterminée sur la base de la résistance électrique (22) de l'élément chauffant (5).

6. Système d'assistance selon la revendication 1, dans lequel le moyen de régulation (14) maintient l'élément chauffant (5) à une température définie ou à une courbe de température définie au-dessus de la température déterminée au moyen du capteur de température (3) et l'unité de calcul (15) obtient comme paramètre de fonctionnement d'élément chauffant la consommation de puissance électrique de l'élément chauffant (5).

**7.** Système d'assistance selon la revendication 1 ou 6, dans lequel est prévu, pour la détection de la température de l'élément chauffant (5), un autre capteur de température (24) couplé thermiquement à l'élément chauffant (5).

**8.** Système d'assistance selon l'une des revendications 1 à 7, dans lequel le capteur de température (3) est disposé sur une paroi interne de la canule (4).

**9.** Système d'assistance (2) vasculaire implantable

   comportant une structure allongée en forme de tube
   comportant une section de canule dans laquelle une canule (4) est réalisée pour le transport de sang depuis un ventricule d'un cœur dans une aorte,
   comportant une section de turbomachine raccordée à la section de canule et dans laquelle est disposée une turbomachine (32) qui sert à transporter du sang depuis un ventricule d'un cœur, à travers la canule (4) et dans une aorte,
   comportant un capteur de température (24, 3) qui est disposé entre un élément chauffant (5) disposé dans la canule (4) et la turbomachine (32) et est espacé spatialement de l'élément chauffant (5),
   dans lequel l'élément chauffant (5) peut provoquer une modification d'une température de fluide dans la canule (4) lorsqu'un courant électrique est passé à travers l'élément chauffant (5), de sorte que celui-ci subit un chauffage par résistance ohmique,
   comportant un dispositif pour l'application d'une impulsion de puissance à l'élément chauffant (5), moyennant quoi une quantité d'énergie définie Ep est introduite dans le volume sanguin de la canule (4), et
   comportant un dispositif pour la mesure de la température du sang s'écoulant à travers la canule (4) au moyen du capteur de température (24, 3), et
   comportant un dispositif pour le calcul du débit volumique Qp sur la base d'un temps de propagation $\Delta t$ détecté pour un maximum de température de la température détectée au moyen du capteur de température (24, 3)
   ou
   comportant un dispositif pour le calcul du débit volumique Qp sur la base d'un temps de propagation $\Delta t$ détecté pour un maximum de température Tm de la température détectée au moyen du capteur de température (24, 3) et d'une hauteur d'amplitude du maximum de température Tm.

**10.** Système d'assistance selon la revendication 9, **ca-** ractérisé en ce que le dispositif de mesure de température présente un autre capteur de température (3) pour la mesure d'une température de référence qui est disposé à une extrémité de la canule (4) opposée à la turbomachine.

**11.** Système d'assistance selon l'une des revendications 1 à 10,
**caractérisé en ce que** l'élément chauffant (5) est réalisé comme un filament chauffant ou un thermofilament.

**12.** Système d'assistance selon l'une des revendications 1 à 11, dans lequel l'élément chauffant (5) est disposé sur une paroi interne de la canule (4).

**Fig. 1a**

**Fig. 1b**

# Fig. 2

# Fig. 3

# Fig. 4

# Fig. 5

**Fig. 6**

**Fig. 7**

Fig. 8

EP 3 801 666 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014141284 A2
- WO 2012112378 A2
- WO 2014165635 A2